# FASCICULE DE BREVET EUROPEEN

(11) **EP 2 931 232 B1**
(45) Date de publication et mention de la délivrance du brevet: **04.10.2017**
(21) Numéro de dépôt: 13818289.4
(22) Date de dépôt: 10.12.2013
(51) Int. Cl.: A61K 8/34, A61K 8/64, A61Q 19/06, A61K 8/97, A61K 8/49

(54) **UTILISATION COSMETIQUE DE L'ASSOCIATION D'UN EXTRAIT DE GERME DE CAROUBE ET DE CAFEINE EN TANT QU'AGENT ACTIF AMINCISSANT**
KOSMETISCHE VERWENDUNG DER KOMBINATION AUS JOHANNISBROTKEIMEXTRAKTEN UND KOFFEIN ALS SCHLANKHEITSWIRKSTOFF
COSMETIC USE OF THE COMBINATION OF A CAROB GERM EXTRACT AND CAFFEINE AS A SLIMMING ACTIVE AGENT

(30) Priorité: 11.12.2012 FR 1203364
(43) Date de publication de la demande: 21.10.2015
(73) Titulaire: ISP Investments Inc., Wilmington, DE 19805 (US)
(72) Inventeur: DOMLOGE, Nouha, F-06560 Valbonne (FR); PORTOLAN, Frédérique, F-06560 Valbonne (FR); CLEMENT, Anne, F-75015 Paris (FR); BOTTO, Jean Marie, F-06560 Garbejaire (FR)
(74) Mandataire: Macquet, Christophe
(86) Numéro de dépôt international: PCT/FR2013/053022
(87) Numéro de publication internationale: WO 2014/091147

(56) Documents cités:
- EP-A1- 0 689 771
- WO-A2-2011/077017
- FR-A1- 2 859 104
- FR-A1- 2 867 683
- US-A1- 2005 186 290
- US-A1- 2008 004 283
- M. HARA-CHIKUMA ET AL: "Progressive Adipocyte Hypertrophy in Aquaporin-7-deficient Mice: ADIPOCYTE GLYCEROL PERMEABILITY AS A NOVEL REGULATOR OF FAT ACCUMULATION", JOURNAL OF BIOLOGICAL CHEMISTRY, vol. 280, no. 16, 1 avril 2005 (2005-04-01), pages 15493-15496, XP055078040, ISSN: 0021-9258, DOI: 10.1074/jbc.C500028200 cité dans la demande
- PARRADO ET AL: "Production of a carob enzymatic extract: Potential use as a biofertilizer", BIORESOURCE TECHNOLOGY, ELSEVIER BV, GB, vol. 99, no. 7, 8 février 2008 (2008-02-08), pages 2312-2318, XP022472753, ISSN: 0960-8524, DOI: 10.1016/J.BIORTECH.2007.05.029 cité dans la demande

## Description

### Domaine de l'invention

La présente invention se situe dans le domaine de la cosmétique et plus particulièrement dans le domaine des méthodes cosmétiques amincissantes. Elle se rapporte à l'utilisation cosmétique de l'association d'un extrait peptidique de germe de caroube (*Ceratonia siliqua L*.) et de caféine ou un de ses dérivés, en tant qu'agent actif amincissant. L'invention se rapporte encore à l'utilisation cosmétique de l'association d'un extrait peptidique de germe de caroube et de caféine ou un de ses dérivés pour augmenter l'expression des aquaglycéroporines et favoriser le déstockage des triglycérides contenus dans les adipocytes.

L'invention a également pour objet une méthode de soin cosmétique comprenant l'application topique, sur au moins une partie de la peau du corps ou du visage, de l'association d'un extrait peptidique de germe de caroube (*Ceratonia siliqua L*.) et de caféine ou un de ses dérivés, dans une composition comprenant un milieu physiologiquement acceptable, pour obtenir un effet amincissant, et plus particulièrement pour diminuer les surcharges adipeuses localisées.

### Arrière plan de l'invention

Le tissu adipeux sous-cutané est situé au niveau de l'hypoderme. C'est une variété de tissu conjonctif où prédominent les adipocytes, organisés en lobules d'environ 5 mm de diamètre, séparés par de fines travées conjonctives. Chaque adipocyte contient une volumineuse vacuole lipidique contenant essentiellement des triglycérides et dont le diamètre peut varier de 40 à 120 µm.

Le tissu adipeux doit être considéré comme un réservoir dynamique, en constant renouvellement, assurant un relai entre les apports alimentaires et les besoins énergétiques de l'organisme. Ainsi, les adipocytes assurent la synthèse, le stockage et la libération des lipides. Ce processus est sous la dépendance d'hormones comme l'insuline ou la leptine.

La synthèse des lipides, ou lipogénèse, s'effectue à partir des triglycérides d'origine alimentaire et du glucose. A l'inverse, les triglycérides stockés dans les adipocytes peuvent être hydrolysés, lors de la lipolyse, pour libérer des acides gras, du glycérol et des mono- et di-esters de glycérol.

Les acides gras non estérifiés ainsi libérés peuvent soit diffuser dans le sang et être alors disponibles pour les besoins énergétiques d'autres cellules de l'organisme, soit être réutilisés rapidement par l'adipocyte pour générer à nouveau des triglycérides par lipogénèse.

Si un déséquilibre durable s'installe dans l'organisme au profit de la lipogénèse, la quantité de lipides stockés dans les adipocytes augmente, conduisant à l'hyperplasie de la masse adipeuse corporelle et plus particulièrement à l'apparition de surcharges adipeuses localisées. En effet, chez l'être humain adulte, sous l'effet des hormones sexuelles, le tissu adipeux se répartit de manière différente selon le sexe et modèle la silhouette. Le tissu adipeux s'accumule sur la poitrine, les hanches, les fesses et les cuisses chez la femme, sur la nuque et les épaules chez l'homme. D'autre part, les surcharges adipeuses localisées sont souvent associées à des modifications de la peau, qui prend un aspect capitonné, dit en «peau d'orange ». Ces surcharges adipeuses localisées sont considérées de nos jours comme inesthétiques, et les personnes concernées peuvent souhaiter améliorer l'aspect de leur peau et de leur silhouette par des méthodes cosmétiques.

De nombreux agents actifs ayant une action sur la lipolyse ou la lipogénèse, avec une visée amincissante ont ainsi été identifiés. Parmi ceux-ci, on peut citer :
- Les bases xanthiques (dérivées de la xanthine), telles que la théophylline, la caféine, la théobromine (décrit dans les brevets FR 2609395, FR 267401), utilisées pour leur action favorisant ainsi l'activité lipolytique des cellules graisseuses.
- Les peptides de synthèse, tels que le peptide de séquence Arg-Gly-Ser-NH₂ (décrit dans le brevet FR 2 858 769), ou encore le peptide de séquence Pro-Leu-Asp-Thr-Ala-Lys-Val-Arg-Leu-Gln (décrit dans le brevet FR 2 879 924) utilisées pour son action dans le découplage entre la réoxydation des coenzymes et la phosphorylation de l'ADP en ATP au niveau des mitochondries.
- Les extraits végétaux, tels que les extraits d'algues marines du genre Palmaria ou Rhodymenia (décrit dans le brevet FR 2 887 447), les extraits de ginkco biloba (voir brevet FR 2 669 537), ou encore les flavones ou isoflavones de soja (décrit dans le brevet WO 01/64177).

Cependant, ces produits présentent généralement une efficacité modérée ou limitée dans le temps. Il est donc important de proposer de nouveaux agents cosmétiques actifs ayant une efficacité remarquable en tant qu'agent actif amincissant.

La solution du problème technique posé réside dans l'utilisation cosmétique de l'association d'un extrait peptidique de germe de caroube et de caféine ou un de ses dérivés. Les inventeurs ont en effet mis en évidence qu'un extrait peptidique de germe de caroube agit sur les aquaglycéroporines, favorisant ainsi le transport du glycérol libéré lors de la lipolyse, hors de l'adipocyte. L'association de cet extrait avec la caféine, ou ses dérivés, cette dernière étant déjà connue pour augmenter la lipolyse, permet d'obtenir un agent actif amincissant aux propriétés remarquables.

Les aquaporines sont une classe de protéines transmembranaires transporteuses d'eau et de petites molécules en solution, entre les cellules et le milieu intérieur. Les aquaporines peuvent être classées en deux sous-groupes distincts : les aquaporines permettant uniquement le transport de l'eau, et les aquaglycéroporines qui permettent, outre le transport de l'eau, le transport du glycérol.

Dans ce deuxième sous-groupe, l'aquaglycéroporine 7 a été identifiée dans la membrane des adipocytes humains et joue un rôle important dans le métabolisme des graisses de réserve (Mariko Hara-Chikuma et al. Progressive adipocyte hypertrophicity in aquaporin-7 deficent mice, J. Biol. Chem, vol.280, n°16, April 22, 2005).

Les propriétés particulières des aquaglycéroporines en font donc des cibles biologiques intéressantes pour favoriser le déstockage des lipides contenus dans les adipocytes.

On entend par déstockage des lipides, le phénomène de lipolyse aboutissant à la sortie du glycérol de la cellule adipocytaire.

L'invention et les avantages qui en découlent seront mieux compris à la lecture de la description.

### Exposé de l'invention

La présente invention a pour premier objet l'utilisation cosmétique de l'association d'un extrait peptidique de germe de caroube (*Ceratonia siliqua L*.) et de caféine ou un de ses dérivés, en tant qu'agent actif amincissant.

L'association de l'extrait peptidique de germe de caroube et de caféine ou un de ses dérivés, utilisée selon l'invention offre notamment les avantages suivants :
- il augmente la lipolyse,
- il augmente l'expression des aquaglycéroporines,
- il favorise le déstockage des lipides et la sortie du glycérol des adipocytes,
- il diminue les surcharges adipeuses localisées,
- il atténue l'aspect en peau d'orange de la peau.

Ainsi, on entend par « agent actif amincissant » au sens de la présente invention, l'association d'un extrait peptidique de germe de caroube et de caféine ou un de ses dérivés, utilisée pour diminuer les surcharges adipeuses localisées, considérées comme inesthétiques et souvent associées à un aspect capitonné, en peau d'orange de la peau.

La graine de caroube (plante du genre Ceratonia), et plus particulièrement la fraction endosperme de cette graine, est largement utilisée pour sa richesse en galactomannanes, dans l'industrie alimentaire sous la dénomination "gomme de caroube". Le germe est la partie la plus riche en protéines de la graine et peut être aisément isolé.

Un extrait protéique de germe de caroube, obtenu par hydrolyse enzymatique, a déjà été décrit (J. Parrado et al., Bioresource Technology 99, 2008). Néanmoins cet extrait est trop concentré en phytohormones, des perturbateurs endocriniens, pour être utilisé en cosmétique.

Pour réaliser l'invention, toute méthode d'extraction ou de purification connue de l'homme du métier peut être utilisée, tel que par exemple le procédé décrit dans la demande de brevet européen EP0689771.

On entend par « extrait peptidique », un mélange de composés majoritairement représentés par des composés de nature peptidique, en solution dans un large volume d'eau ou d'autres solvants, polaires ou un mélange de ces solvants.

On entend par « composés de nature peptidique », les fragments de protéines et les peptides présents dans l'extrait peptidique selon l'invention.

On entend par « application topique », le fait d'appliquer ou d'étaler l'agent actif selon l'invention, ou une composition le contenant, à la surface de la peau ou d'une muqueuse.

On entend par « physiologiquement acceptable », tout composé approprié pour entrer en contact avec la peau ou une muqueuse sans provoquer de réactions de toxicité ou d'intolérance.

On entend par « surcharge adipeuse localisée » une zone hypertrophiée du tissu adipeux sous cutané, qui peut avoir un aspect en peau d'orange.

Dans la suite de l'exposé, on utilisera indifféremment les termes « agent actif amincissant » et « association d'un extrait peptidique de germe de caroube et de caféine ou un de ses dérivés ».

L'extrait peptidique de germe de caroube selon l'invention peut être obtenu par extraction de protéines d'origine végétale, suivie d'une hydrolyse contrôlée qui libère les composés de nature peptidique biologiquement actifs.

L'utilisation d'extraits peptidiques, et en particulier d'extraits peptidiques de bas poids moléculaires, présente de nombreux avantages en cosmétique. Outre le fait de générer des composés de nature peptidique qui ne préexistaient pas dans le mélange protéique de départ, l'hydrolyse et la purification permettent d'obtenir des mélanges plus stables, des compositions plus facilement reproductibles et ne provoquant pas de réactions allergiques en cosmétique.

Pour réaliser l'extraction, on utilise les germes de graines de caroube (plante du genre Ceratonia). Toute méthode d'extraction ou de purification connue de l'homme du métier peut être utilisée afin de préparer l'extrait selon l'invention. Par exemple, l'hydrolyse ménagée permet de dégager des composés de nature peptidique. Il est possible, mais non nécessaire pour réaliser l'invention, soit d'extraire les protéines concernées puis de les hydrolyser, soit d'effectuer l'hydrolyse sur un extrait brut puis de purifier les composés de nature peptidique ensuite.

Dans une première étape, les germes contenus dans les graines sont broyés afin d'obtenir une poudre ou farine. La poudre ainsi obtenue peut être préalablement traitée par une cellulase afin de favoriser l'élimination des sucres, et en particulier des polysaccharides insolubles.

On réalise ensuite l'extraction des protéines du germe suivant le procédé classique (Osborne, T. B., The Vegetable Proteins, 2nd Edition. Longmans, Green and Co., London, 1924) modifié ; le broyat de germe de caroube est mis en suspension dans une solution alcaline contenant un produit adsorbant de type polyvinylpolypyrrolidone (PVPP) insoluble (0,01 - 20 %) ; en effet, il est connu que les hydrolyses et les purifications ultérieures sont facilitées par ce moyen. En particulier, la concentration en substances de type phénoliques, interagissant avec les protéines, se trouve nettement réduite. Les protéines peuvent ensuite être précipitées en faisant varier la force ionique ou en acidifiant le milieu, ce qui permet d'éliminer les composants solubles et les acides nucléiques. Le précipité est ensuite lavé à l'aide d'un solvant organique tel que, par exemple, l'éthanol ou le méthanol, puis le solvant est évaporé par séchage sous vide. Le précipité riche en protéines est remis en solution dans l'eau ou un autre solvant et constitue alors une forme plus purifiée de l'extrait.

L'extraction peut également être réalisée en milieu neutre ou acide toujours en présence de polyvinylpolypyrrolidone. Après une étape de filtration, l'étape de précipitation s'effectue alors à l'aide d'un agent classique de précipitation tel que les sels (chlorure de sodium, sulfate d'ammonium) ou un solvant organique (alcool, acétone). Le précipité obtenu peut être séparé des agents de précipitation par dialyse après remise en solution dans de l'eau ou un autre solvant.

La fraction soluble, contenant les protéines, les glucides et éventuellement des lipides, est recueillie après des étapes de centrifugation et de filtration. Cette solution brute est ensuite hydrolysée dans des conditions ménagées pour générer des peptides solubles. L'hydrolyse se définit comme étant une réaction chimique impliquant le clivage d'une molécule par de l'eau, cette réaction pouvant se faire en milieu neutre, acide ou basique. Selon l'invention, l'hydrolyse est réalisée par voie chimique et/ou de façon avantageuse par des enzymes protéolytiques parmi lesquelles on peut alors citer les endoprotéases d'origine végétale (papaïne, bromelaïne, ficine).

Selon un premier mode de réalisation de l'invention, l'agent actif amincissant comprend l'extrait peptidique de germe de caroube hydrolysé obtenu à cette étape.

Pour les mêmes raisons que précédemment, c'est-à-dire l'élimination des substances polyphénoliques, une quantité de polyvinylpolypyrrolidone peut être additionnée au milieu réactionnel lors de cette étape d'hydrolyse ménagée. L'extrait obtenu peut être encore purifié afin de sélectionner les composés de nature peptidiques de bas poids moléculaires. Le fractionnement peut s'effectuer avantageusement par ultrafiltration et/ ou par une méthode de type chromatographique.

On procède ensuite à une phase de dilution dans de l'eau ou dans tout mélange de solvants contenant de l'eau. Ainsi, selon une forme avantageuse de l'invention, l'extrait de germe de caroube selon l'invention est avantageusement dilué dans un ou plusieurs solvants physiologiquement acceptables, tels que l'eau, le glycérol, l'éthanol, le propanediol, le butylène glycol, le dipropylène glycol, les diglycols éthoxylés ou propoxylés, les polyols cycliques ou tout mélange de ces solvants. L'extrait de germe de caroube dilué est ensuite stérilisé par ultrafiltration.

Après cette dilution, on obtient un extrait peptidique caractérisé par un poids sec de 2 à 5 g/Kg, une concentration en composés de nature peptidique de 1 à 10 g/l, préférentiellement de 1,5 à 3,5 g/l, une concentration en sucres de 0,05 à 1 g/l, préférentiellement de 0,1 à 0,3 g/l et une concentration en polyphénols inférieure à 1 % par rapport au poids sec.

Ainsi, selon une forme avantageuse de l'invention, l'extrait de germe de caroube à un poids sec de 2,5 g/kg et contient entre 1,5 et 3,5 g/l de composés de nature peptidique.

Les caractéristiques physico-chimiques et la teneur en composés de nature protéique et peptidique de l'extrait obtenu selon l'invention sont analysées qualitativement et quantitativement, selon les techniques classiques bien connues de l'homme du métier.

L'extrait obtenu est composé de peptides de poids moléculaire inférieur à 5 kDa et est caractérisé par une concentration en sucres inférieure à 15 % et une concentration en polyphénols inférieure à 1 % par rapport au poids sec.

Ainsi, selon une forme avantageuse de l'invention, l'extrait de germe de caroube est un extrait peptidique dans lequel les composés de nature peptidique ont un poids moléculaire inférieur à 5 kDa.

La caféine est une molécule hétérocyclique d'origine végétale faisant partie du groupe des bases puriques, et plus particulièrement des méthylxanthines.

L'effet lipolytique de la caféine a été démontré par de nombreuses études *in vitro* et *in vivo.* La caféine favorise l'accumulation d'AMP cyclique qui lui-même active la triglycéride-lipase, responsable au sein de l'adipocyte de la transformation des triglycérides en glycérol et acides gras libres.

Les dérivés de caféines ont été développés pour améliorer la solubilité, la pénétration cutanée, la biodisponibilité ou l'efficacité de la caféine. Les dérivés de caféine peuvent être choisis parmi les dérivés acides de caféine, les sels de caféine, et plus particulièrement les sels métalliques de carboxylate de caféine.

La présente invention a également pour objet l'utilisation de l'association d'un extrait peptidique de germe de caroube et de caféine ou un de ses dérivés pour augmenter l'expression des aquaglycéroporines et plus particulièrement de aquaglycéroporine 7.

L'activité moléculaire caractéristique de l'invention est définie *in vitro* par la capacité de l'agent actif amincissant à augmenter l'expression des aquaglycéroporines, soit par l'augmentation de la synthèse protéique des aquaglycéroporines (par modulation directe ou indirecte de l'expression génique des aquaglycéroporines), soit par d'autres processus biologiques tels que la stabilisation de la protéine aquaglycéroporine ou encore la stabilisation des transcrits d'ARN messager.

Préférentiellement, selon la présente invention, l'aquaglycéroporine est l'aquaglycéroporine 7.

La présente invention a également pour objet l'utilisation de l'association d'un extrait peptidique de germe de caroube et de caféine ou un de ses dérivés pour augmenter la lipolyse et favoriser le déstockage des lipides et la sortie du glycérol des adipocytes.

L'activité biologique caractéristique de l'invention est définie *in vitro* par la capacité de l'agent actif amincissant à diminuer la taille et le nombre de gouttelettes lipidiques dans les adipocytes.

La présente invention a encore pour objet une méthode de soin cosmétique comprenant l'application topique sur au moins une partie de la peau du corps ou du visage, de l'association d'un extrait peptidique de germe de caroube (*Ceratonia siliqua L*.) et de caféine ou un de ses dérivés, dans une composition comprenant un milieu physiologiquement acceptable, pour obtenir un effet amincissant, et plus particulièrement pour diminuer les surcharges adipeuses localisées.

La présente invention a encore pour objet une méthode de soin cosmétique comprenant l'application topique sur au moins une partie de la peau du corps ou du visage, de l'association d'un extrait peptidique de germe de caroube (*Ceratonia siliqua L*.) et de caféine ou un de ses dérivés, dans une composition comprenant un milieu physiologiquement acceptable, pour atténuer l'aspect en peau d'orange de la peau.

Avantageusement, l'extrait peptidique de germe de caroube est présent à une concentration comprise entre 0,0001 % et 20 % du poids total de la composition, et préférentiellement à une concentration comprise entre 0,05 % et 5 % du poids total de la composition, dans un milieu physiologiquement acceptable.

Avantageusement, la caféine, ou un de ses dérivés, est présente à une concentration comprise entre 0,0001 % et 20 % du poids total de la composition, et préférentiellement à une concentration comprise entre 0,05 % et 5 % du poids total de la composition, dans un milieu physiologiquement acceptable.

Selon un autre mode de réalisation avantageux de l'invention L'extrait peptidique
de germe de caroube peut être encapsulé ou inclus dans un vecteur cosmétique tels que les liposomes ou toute autre microcapsule utilisée dans le domaine de la cosmétique ou adsorbé sur des polymères organiques poudreux, des supports minéraux comme les talcs et bentonites.

Les compositions pour la mise en oeuvre de l'invention pourront notamment se présenter sous forme d'une solution aqueuse, hydro-alcoolique ou huileuse; d'une émulsion huile-dans-eau, eau-dans-huile ou émulsions multiples ; elles aussi peuvent se présenter sous forme de suspensions, ou encore poudres, adaptées à une application sur la peau, les muqueuses, les lèvres et/ou les cheveux.

Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol.

Ces compositions peuvent comprendre, en outre, tout additif communément utilisé dans le domaine d'application envisagé ainsi que les adjuvants nécessaires à leur formulation, tels que des solvants, des épaississants, des diluants, des antioxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Avantageusement, la composition utilisable pour réaliser l'invention peut comprendre, outre l'agent actif amincissant selon l'invention, au moins un autre agent actif présentant des effets cosmétiques similaires et/ou complémentaires à ceux de l'invention. Selon l'invention, cet agent actif sera défini comme un « agent actif additionnel ».

Par exemple, le ou les agents actifs additionnels peuvent être choisi parmi : les agents anti-âges, raffermissants, éclaircissants, hydratants, drainants, favorisant la microcirculation, agents pharmaceutiques, exfoliants, desquamants, stimulant la matrice extracellulaire, activant le métabolisme énergétique, antibactériens, antifongiques, apaisants, anti-radicalaires, anti-UV, anti-acnés, anti-inflammatoires, anesthésiques, procurant une sensation de chaleur, procurant une sensation de fraicheur, amincissants.

De tels agents additionnels peuvent être choisis dans les groupes comprenant :
- la vitamine A et notamment l'acide rétinoïque, le rétinol, le rétinol proprionate, le rétinol palmitate,
- la vitamine B3 et plus particulièrement le niacinamide, le niconitate de tocophérol,
- la vitamine B5, la vitamine B6, la vitamine B12,
- la vitamine C, notamment l'acide ascorbique, l'ascorbyl glucoside, l'ascorbyl tetrapalmitate, magnesium et sodium ascorbyl phosphate,
- les vitamines E, F, H, K, PP, le coenzyme Q10,
- les inhibiteurs de métalloproteinase, ou un activateur des TIMP,
- la DHEA, ses précurseurs et dérivés,
- les acides aminés tels que l'arginine, ornithine, hydroxyproline, hydroxyproline dipalmitate, palmitoylglycine, hydroxylysine, méthionine et ses dérivés, composés acides aminés N-acyl,
- les peptides naturels ou de synthèse, incluant les, di-, tri-, tetra-, penta- et hexapeptides et leurs dérivés lipophiles, isomères et complexés avec d'autres espèces telles qu'un ion métal (e.g. cuivre, zinc, manganèse, magnésium, et autres). Par exemple les peptides commercialement connus sous le nom MATRIXYL^{®}, ARGIRELINE^{®}, COLLAXYL™, PEPTIDE VINCI 02™, CHRONOGEN™, LAMINIXYL IS™, PEPTIDE Q10™,
- les extraits peptidiques végétaux tels que extraits de soja, d'épeautre, de vigne, de colza, de lin, de riz, de maïs, de pois,
- les extraits de levures, les extraits d'Artemia Salina,
- l'acide déhydroacétique (DHA),
- les phystostérols d'origine synthétique ou naturelle,
- l'acide salicylique et ses dérivés, les alpha- et bêta-hydroxyacides,
- les sucres aminés, glucosamine, D-glucosamine, N-acetyl- glucosamine, N-acetyl-D-glucosamine, mannosamine, N-acetyl mannosamine, galactosamine, N-acetyl galactosamine,
- les extraits de polyphénols, isoflavones, flavonoïdes, tels que les extraits de raisin, les extraits de pin, les extraits d'olives,
- les lipides tels que les céramides ou les phospholipides, les huiles d'origine animale, telles que le squalène ou le squalane ; les huiles végétales, telles que l'huile d'amandes douces, de coprah, de ricin, de jojoba, d'olive, de colza, d'arachide, de tournesol, de germes de blé, de germes de maïs, de soja, de coton, de luzerne, de pavot, de potiron, d'onagre, de millet, d'orge, de seigle, de carthame, de passiflore, de noisette, de palme, de noyau d'abricot, d'avocat, de calendula ; les huiles végétales éthoxylées, le beurre de karité,
- Tous écrans UV et filtres solaires,

De façon particulièrement avantageuse, l'invention peut comprendre au moins un agent actif additionnel connus pour son action amincissante, inhibant la lipogénèse ou stimulant la lipolyse, tels que : l'AMP cyclique et ses dérivés, les agents activateurs de l'enzyme adénylate cyclase et les agents inhibiteurs de l'enzyme phosphodiestérase, l'extrait de centella asiatica, l'asiaticoside et l'acide asiatique, les méthyls xanthines, la théine, la théophylline, la théobromine, la forskoline, l'esculine et l'esculoside, les inhibiteurs d'ACE, le peptide Val-Trp, l'inhibiteurs du neuropeptide Y, l'enkephaline, l'extrait de gingko biloba, l'extrait de dioscorea, la rutine, l'extrait de yerba mate, l'extrait de guarana, les oligosaccharides, les polysaccharides, la carnitine, l'extrait de lierre, l'extrait de fucus, l'extrait hydrolysé de Prunella vulgaris, l'extrait hydrolysé de Celosia cristata, l'extrait d'Anogeissus leiocarpus, l'extrait de feuilles de Manihot utilissima, la palmitoylcarnitine, la carnosine, la taurine, l'extrait de sureau, les extraits d'algue tel que l'extrait de Palmaria Palmata, le peptide de synthèse de séquence Arg-Gly-Ser-NH₂, commercialisé sous le nom ATPeptide™, le peptide de synthèse de séquence Pro-Leu-Asp-Thr-Ala-Lys-Val-Arg-Leu-Gln commercialisé sous le nom ATPeptide™.

La composition utilisable selon l'invention pourra être appliquée par toute voie appropriée, notamment orale, ou topique externe, et la formulation des compositions sera adaptée par l'homme du métier.

Avantageusement, les compositions selon l'invention se présentent sous une forme adaptée à l'application par voie topique. Ces compositions doivent donc contenir un milieu physiologiquement acceptable, c'est-à-dire compatible avec la peau et les phanères, et couvrent toutes les formes cosmétiques.

Il est bien évident que l'invention s'adresse aux mammifères en général, et plus particulièrement aux êtres humains.

Des modes de réalisation particuliers de ce procédé de traitement cosmétique résultent également de la description précédente. D'autres avantages et caractéristiques de l'invention apparaîtront mieux à la lecture des exemples donnés à titre illustratif et non limitatif.
Figure 1 : Immuno-détection de l'aquaglycéroporine 7 dans les cellules 3T3-L1.
Figure 2 : Quantification de la taille des gouttelettes lipidiques dans des cellules 3T3-L1.

### Exemple 1 : Préparation d'un extrait peptidique de caroube (Ceratonia siliqua L.)

Le germe de caroube (*Ceratonia siliqua L*.) sous forme de poudre est mis en solution dans 70 volumes d'eau et le pH est ajusté à une valeur comprise entre 4,5 et 5,5.

Afin d'éliminer les sucres insolubles, une hydrolyse à l'aide d'une cellulase est réalisée. Pour cela, 2 % de celluclast CL® (Novozymes) et 2 % de POLYCLAR® 10 (polyvinylpyrrolidone - PVPP - insoluble) sont ajoutés dans le milieu réactionnel. Le milieu réactionnel est ensuite chauffé deux heures à 50°C puis désactivé une heure à 80°C. Une étape de filtration permet d'écarter le filtrat riche en carbohydrates pour ne conserver que le résidu solide.

Ce dernier est caractérisé par une teneur en protéines comprise entre 45 et 50 % et un taux de sucres compris entre 20 et 30 %.

Le résidu sec ainsi obtenu est mis en solution dans 100 volumes d'eau en présence de 2 % de POLYCLAR® 10. Le mélange est ajusté à un pH compris entre 8,0 et 8,5 avec une solution aqueuse de soude à 2 M.

Afin d'améliorer l'extraction des protéines, une première hydrolyse est réalisée à l'aide de 2 % d'Alcalase® (Novozymes). L'hydrolyse est obtenue après 2 heures, sous agitation, à 55°C. On procède à l'inactivation de l'enzyme en chauffant la solution à 80°C pendant 2 heures. Après désactivation, le mélange réactionnel est filtré et le filtrat est recueilli. Il s'agit de l'extrait protéique intermédiaire de germe de caroube.

A ce stade de la préparation, les composés de nature peptidique et protéique de ce filtrat sont caractérisés par électrophorèse sur gel de polyacrylamide (gels NuPAGE® Bis-Tris Pre-cast, Invitrogen). Pour cela, le filtrat est chauffé à 70°C pendant 10 minutes dans des conditions réductrices dénaturantes dans un tampon de préparation d'échantillon NuPAGE® LDS. Une solution de NuPAGE® antioxydante est ajoutée dans la cuve intérieure (cathode) pour éviter que les protéines réduites ne se réoxydent durant l'électrophorèse. La migration des protéines est réalisée dans du tampon de migration NuPAGE® MES en présence d'un standard de poids moléculaire (SeeBlue Plus2). La coloration des protéines est effectuée à l'aide de Bleu de Coomassie® R-250. Le profil protéique ainsi obtenu montre que les composés de nature peptidique et protéique du filtrat ont des poids moléculaires compris entre 50 et 10 kDa.

L'extrait protéique intermédiaire de germe de caroube est ensuite mis en solution dans 100 volumes d'eau en présence de 2 % de POLYCLAR® 10. Le mélange est ajusté à un pH compris entre 4 et 5 avec une solution aqueuse d'acide chlorhydrique 1 M.

Une étape d'hydrolyse des protéines est alors réalisée à l'aide d'une endoprotéase. Pour cela, de la bromélaïne à 2 % est ajoutée dans le milieu réactionnel. L'hydrolyse est obtenue après 2 heures d'agitation à 50°C. On procède à l'inactivation de l'enzyme en chauffant la solution à 80°C pendant 2 heures.

La purification de l'extrait ainsi obtenu se poursuit par des filtrations successives à l'aide de filtres-plaques Seitz-Orion de porosité décroissante (jusqu'à 0,2 µm) afin d'obtenir une solution brillante et limpide. Après cette série de filtration, l'extrait de germe de caroube est caractérisé par un poids sec compris entre 20 et 25 g/kg, un taux de protéines compris entre 10 et 15 g/l, un taux de sucres compris entre 5 et 6 g/l, un taux d'acides aminés compris entre 1 et 2 g/l et un taux de polyphénols totaux compris entre 0,5 et 1 g/l. Les protéines sont dosées par une méthode colorimétrique spécifique (méthode de Lowry)

Le profil protéique de cet extrait est analysé par gel d'électrophorèse. Dans les mêmes conditions que décrites précédemment, on observe 2 grandes familles de protéines : la 1ère famille, minoritaire, correspond à des protéines de poids moléculaire de 25 à 20 kDa et la 2ème famille, très majoritaire, correspond à des protéines de poids moléculaire inférieur à 5kDa.

Cet extrait est alors purifié en éliminant les protéines de poids moléculaire supérieur à 5 kDa par des étapes de filtrations à flux tangentiel. Pour cela, l'extrait de germe de caroube est pompé sous pression à travers un support Pellicon® équipée de cassette Pellicon® 2 Biomax 30 kDa. Le premier filtrat obtenu est récupéré pour être de nouveau filtré à travers une autre cassette Pellicon® 2 Biomax 5 kDa.

En fin de purification, on obtient un extrait de germe de caroube jaune-orangé, brillant et limpide. Il est caractérisé par un poids sec de compris entre 8 et 9 g/kg, une teneur en protéines comprise entre 6 et 7 g/l, un taux de sucres entre 0,3 et 0,5 g/l et un taux de polyphénols totaux inférieur à 0,1 g/l.

On procède alors à une phase de dilution dans un mélange eau-glycérol afin d'obtenir un extrait peptidique caractérisé par un poids sec de 2 à 5 g/kg, et préférentiellement de 2,5 g/kg, une concentration en composés de nature peptidique de 1,5 à 3,5 g/l, une concentration en sucres de 0,1 à 0,3 g/l (soit inférieure à 15 %) et une concentration en polyphénols inférieure à 1 %.

Cet extrait purifié et dilué correspond à l'extrait peptidique de germe de caroube selon l'invention. Il est caractérisé par le fait que les composés de nature peptidique ont un poids moléculaire inférieur à 5 kDa, que la teneur en polyphénols est inférieure à 1 %.

Cette solution est alors analysée en chromatographie liquide haute pression à l'aide d'un appareil HP1100 piloté par le logiciel ChemStation. La colonne utilisée lors de l'élution de l'extrait de caroube est une Nucleosil® 300-5 C4 MPN (125 x 4 mn). Cette colonne permet d'analyser en chromatographie des protéines ayant des poids moléculaires de 0,2 à 25 kDa (selon un gradient de solvants approprié). Dans ces conditions chromatographiques, il a été isolé plusieurs fractions peptidiques. Ces diverses fractions ont été analysées par spectrométrie de masse afin d'identifier leurs pics moléculaires. La détermination de la composition en acides aminés a également été réalisée. Celle-ci est obtenue après hydrolyse acide et identification par chromatographie liquide haute pression à l'aide d'une pré-dérivation au PICT (phényl-isothiocyanate).

### Exemple 2 : Evaluation de l'expression de l'aquaglycéroporine 7

Le but de cette étude est de déterminer l'influence de l'association de l'extrait de caroube selon l'exemple 1 et de caféine, sur l'expression de l'aquaglycéroporine 7 dans des cellules adipocytaires différenciées 3T3-L1.

### Protocole :

Culture et différenciation des cellules 3T3-L1 :
Les cellules adipocytaires 3T3-L1 sont cultivées en milieu DMEM 4,5 g/l glucose, 2 mM Glutamine et 10 % de sérum de veau foetal.

2 jours après l'entrée en phase de confluence cellulaire, la différenciation des cellules 3T3-L1 en adipocytes est induite par l'addition d'une solution de 0,5 mM d'IBMX, 1 µM de dexaméthasone et 10 µg/ml d'insuline (Sigma, St Louis, MO, USA) dans le milieu de culture pendant 3 jours. Ensuite, seule l'insuline est maintenue pendant 3 à 4 jours de culture supplémentaire. Les cellules sont ensuite maintenues en culture, en milieu standard, pendant encore 3 jours.

### Traitement :

Le traitement est réalisé dès le début de la phase d'induction de la différenciation par une application quotidienne, pendant 12 jours, soit de 1X PBS (Lonza, Rockland USA) pour le contrôle non traité, soit d'extrait de germe de caroube de poids sec 2,5 g/kg, tel qu'obtenu à l'exemple 1, dilué à 1 % dans du PBS, soit d'extrait de germe de caroube, tel qu'obtenu à l'exemple 1, dilué à 1 % dans du PBS puis ajout de caféine à 2mM pendant les 5 dernières heures du traitement. Un traitement de 5 heures avec de la caféine à 2 mM a également été réalisé.

### Immuno-marquage de l'aquaglycéroporine 7 :

Les cellules sont lavées 3 fois par du 1X PBS (Lonza, Rockland, USA) et fixées par 3,7 % de formaldéhyde (Sigma Aldrich, USA) pendant 10 min à température ambiante. Les membranes cellulaires sont perméabilisées par de l'acetone pendant 4 min à -20°C. Les sites non spécifiques sont saturés par de la serum albumine bovine à 3 % (Sigma-Aldrich, Steinheim, Germany), pendant 15 min. L'anticorps primaire (rabbit polyclonal anti-aquaglycéroporine 7, Santa Cruz Biotechnology) diluted at 1/100) est appliqué pendant 1,5 heure. Après plusieurs rinçages, un second anticorps, couplé à la sonde Alexa Fluor 488 (Alexa Fluor 488 donkey anti-rabbit (Invitrogen, Fisher) diluée au 1/1000) est appliqué pendant 1 heure. Les lames sont ensuite montées dans du Fluoromount G (Electron Microscopy Science, Hatfield, UK).

Les cellules sont examinées au microscope Nikon Eclipse 80i, à l'objectif 40X, et les photos sont réalisées à l'aide d'un appareil Nikon Digital DXM1200C.

3 images par condition sont analysées à l'aide du logiciel Image-Pro Analyzer 6.3. La somme des intensités lumineuses est ajustée par rapport à la surface de culture (Mc Mullen and coll., 2010).

L'analyse statistique utilise un test t-Student pour données non appariées, dans lequel P<0,05 est considéré comme significatif; P<0,01 comme très significatif, et P<0,005 comme hautement significatif.

### Résultats :

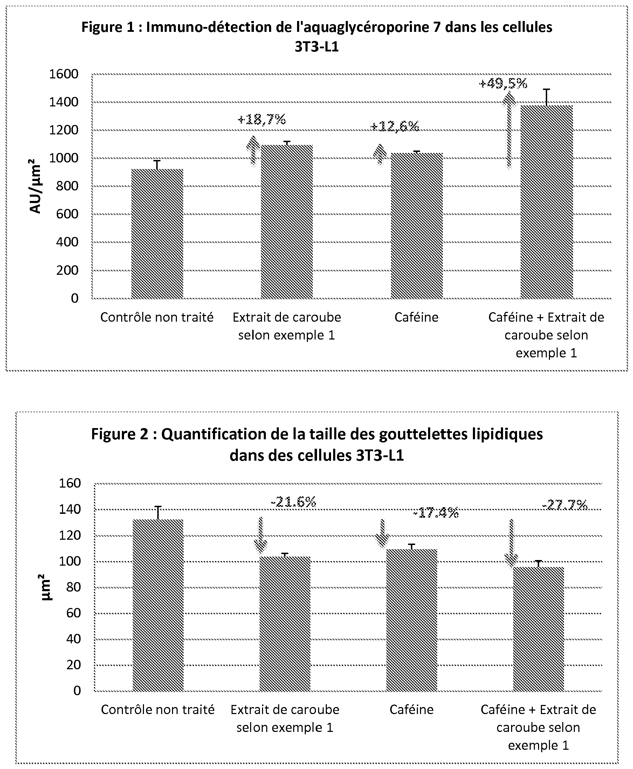

Les observations microscopiques montrent une augmentation non significative de 12,6 % de la fluorescence dans les cellules 3T3-L1 différenciées traitées par de la caféine, par rapport au contrôle non traité. On observe également une augmentation significative de 18,7 % de la fluorescence dans les cellules traitées par l'extrait de caroube selon l'exemple 1 à 1 %, par rapport au contrôle non traité. Les analyses quantitatives montrent enfin une augmentation très significative de 49,5 % de la fluorescence dans les cellules par rapport au contrôle non traité, comme cela est illustré par la figure 1.

### Conclusions :

L'association d'extrait de caroube selon l'exemple 1 et de caféine provoque une augmentation très significative de l'expression de l'aquaglycéroporine 7 dans des cellules adipocytaires différenciées 3T3-L1. La caféine potentialise très sensiblement l'effet de l'extrait de caroube selon l'exemple 1 à 1 %.

### Exemple 3 : Evaluation des gouttelettes lipidiques contenues dans les adipocytes

Le but de cette étude est de mesurer l'influence de l'association de l'extrait de caroube selon l'exemple 1 et de caféine, sur la taille et le nombre de gouttelettes lipidiques contenues dans les cellules adipocytaires différenciées 3T3-L1.

### Protocole :

La culture, la différenciation des cellules 3T3-L1, puis le traitement par les composés à tester sont réalisés comme dans l'exemple 2.

Détection des lipides par le Rouge du Nil :
La détection des lipides est réalisée à l'aide du colorant fluorescent Rouge du Nil, une phénoxazone qui marque intensément les lipides intracellulaires

La couleur de la fluorescence observée est directement dépendante de l'hydrophobicité du milieu environnant. Cette propriété spécifique du Rouge du Nil permet de différencier les lipides neutres, marqués en jaune doré, des phospholipides, marqués en rouge.

Les cellules sont fixées par une solution de formaldéhyde à 3,7 % pendant 10 minutes, puis marquées par une solution de Rouge du Nil à 100 nM dans du PBS pendant 10 minutes, et enfin rincées dans du PBS.

Les cellules sont examinées au microscope à fluorescence. La quantification et l'analyse statistique sont réalisées de la même manière qu'à l'exemple 2.

### Résultats :

L'intensité du marquage au Rouge du Nil, la taille et le nombre des gouttelettes dans les cellules 3T3-L1 différenciées traitées par l'extrait de caroube selon l'exemple 1 à 1 %, par la caféine à 2mM, et par l'association d'extrait de caroube selon l'exemple 1 et de caféine sont diminués par rapport aux cellules non traitées.

Concernant l'association d'extrait de caroube selon l'exemple 1 et de caféine, les analyses quantitatives montrent une diminution significative de 42,78 % de l'intensité du marquage, une diminution de 27,7 % de la taille des gouttelettes et une diminution significative de 33 % du nombre de gouttelettes par rapport aux cellules non traitées. La quantification de l'intensité de la coloration, de la taille et du volume des gouttelettes lipidiques est illustrée par les tableaux 1, 2 et 3. La quantification de la taille des gouttelettes lipidiques est illustrée par la figure 2.

| Tableau 1 : Intensité du marquage au Rouge du Nil | Ratio (Pixels/ µm²) | % de diminution | Déviation standard | Test t-Student |
|---|---|---|---|---|
| Contrôle non traité | 288.10 | | 31.22 | |
| Extrait de caroube selon l'exemple 1 à 1 % | 185.44 | -35.63 | 11.76 | significatif |
| Caféine | 180.47 | -37.36 | 9.22 | significatif |
| Association extrait de caroube selon l'exemple 1 et caféine 2mM | 164.85 | -42.78 | 19.47 | significatif |

| Tableau 2 : Evaluation de la taille des gouttelettes | Taille moyenne des gouttelettes ((µm²) | % de diminuti on | Déviation standard | Test t-Student |
|---|---|---|---|---|
| Contrôle non traité | 132.65 | | 9.76 | |
| Extrait de caroube selon l'exemple 1 à 1 % | 104.01 | -21.60 | 2.47 | Significatif |
| Caféine | 109.55 | -17.40 | 3.75 | Significatif |
| Association extrait de caroube selon l'exemple 1 et caféine 2mM | 95.92 | -27.7 | 4.74 | Non significatif |

| Tableau 3 : Evaluation du Nombre de gouttelettes | Nombre de gouttelettes | % de diminution | Déviation standard | Test t-Student |
|---|---|---|---|---|
| Contrôle non traité | 266 | | 29,00 | |
| Extrait de caroube selon l'exemple 1 à 1 % | 189 | -29 | 20 | significatif |
| Caféine | 219 | -18 | 13 | Non significatif |
| Association extrait de caroube selon l'exemple 1 et caféine 2mM | 178 | -33 | 22 | significatif |

### Conclusion :

L'association d'extrait de caroube selon l'exemple 1 et de caféine provoque une diminution significative de la taille et du nombre de gouttelettes lipidiques dans des cellules adipocytaires différenciées 3T3-L1.

### Exemple 4 : Evaluation clinique n° 1 de l'effet amincissant de l'association d'extrait de caroube selon l'exemple 1 et de caféine

Le but de cette étude est d'évaluer l'effet amincissant de l'association de l'extrait de caroube selon l'exemple 1 et de caféine, chez l'homme, par des mesures centimétriques.

### Protocole :

Une étude clinique a été réalisée sur 39 volontaires, ayant un âge compris entre 34 et 63 ans (âge moyen : 52 ans), par application biquotidienne sur les différentes zones à tester (taille, ventre, hanches, fesses, cuisses), pendant une durée de 28 jours.

Le produit testé est une composition cosmétique standard comprenant 2 % de caféine et 2 % d'extrait de caroube selon l'exemple 1.

L'évaluation de l'efficacité se fait par comparaisons des mesures à T0 et à T 28 jours, chaque volontaire étant son propre témoin.

### Résultats :

Dans les conditions expérimentales, après 28 jours d'application, on observe une diminution moyenne significative des mesures centimétriques sur les cuisses (0,8 cm en moyenne), sur la taille (-0,6 cm en moyenne) et sur le ventre (-0,9 cm en moyenne).

La série de données suit une loi normale. L'analyse statistique utilise un test t-Student pour données appariées, dans lequel P<0,05 est considéré comme significatif et noté *.

| Tableau 4 | T0 | T 1 mois | Variation moyenne (T0-T1 mois) cm | P significat if si p<0,05 | % de sujet réponda nt | Variation du meilleur tiers (cm) | % de sujet répondant sur au moins une zone |
|---|---|---|---|---|---|---|---|
| Cuisse traitée | 57,16 | 56,19 | -0,8* | 3,16^{E-08} | 69 % | -1,48 | 88 % |
| Cuisse témoin | 57,13 | 56,95 | | | | | |
| Ventre (cm) | 94,77 | 93,84 | -0,9* | 6,03^{E-05} | 62 % | -2,05 | |
| Taille (cm) | 80,75 | 80,19 | -0,6* | 1,56^{E-02} | 59 % | -1,50 | |

### Conclusion :

L'application biquotidienne de l'association de l'extrait de caroube selon l'exemple 1 et de caféine à 2 % pendant 1 mois, permet un amincissement significatif des cuisses, de la taille et du ventre.

### Exemple 5 : Evaluation clinique n° 2 de l'effet amincissant de l'association d'extrait de caroube selon l'exemple 1 et de caféine

Le but de cette étude est d'évaluer l'effet amincissant de l'association de l'extrait de caroube selon l'exemple 1 et de caféine, chez l'homme, par des mesures centimétriques.

### Protocole :

Une étude clinique a été réalisée sur 26 volontaires, ayant un âge compris entre 30 et 65 ans (âge moyen : 43,7 ans), par application biquotidienne sur les différentes zones à tester (taille, ventre, hanches, fesses, cuisses), pendant une durée de 28 jours.

Le produit testé est une composition cosmétique standard comprenant 2 % de caféine et 2 % d'extrait de caroube selon l'exemple 1.

L'évaluation de l'efficacité se fait par comparaisons des mesures à T0 et à T 28 jours, chaque volontaire étant son propre témoin.

### Résultats :

Dans les conditions expérimentales, après 28 jours d'application, on observe une diminution moyenne significative de - 2,2 cm de la circonférence de l'abdomen, une diminution moyenne significative de - 1,2 cm de la circonférence supérieure de la cuisse, et une diminution moyenne significative du volume des troncs de cuisses.

La série de données suit une loi normale. L'analyse statistique utilise un test t-Student pour données appariées, dans lequel P<0,05 est considéré comme significatif.

D'autre part, un questionnaire a permis d'établir que la majorité des volontaires estimait les zones traitées plus lisse et plus ferme et l'aspect peau d'orange atténué.

### Conclusion :

L'application biquotidienne de l'association de l'extrait de caroube selon l'exemple 1 et de caféine à 2 % pendant 1 mois, permet un amincissement significatif des cuisses et du ventre, ainsi qu'une atténuation de l'aspect peau d'orange.

### Exemple 6 : Préparation de compositions

### 1 Crème amincissante 1

| ***Phase*** | ***Composant*** | **% *massique*** |
|---|---|---|
| A | Eau purifiée | 38,84 |
| A | Methylpropane diol | 3,00 |
| A | Glycereth-26 | 3,00 |
| B | Glycérine 100 % veg. | 2,00 |
| B | Gomme de xanthane standard | 0,3 |
| C | Isododecane | 15,00 |
| C | DC 1503 Fluid | 3,0 |
| C | Acetate de tocophérol | 0,5 |
| D | Pemulen TR1 | 0,5 |
| D | Synthalen K | 0,3 |
| E | NA 4 EDTA | 0,1 |
| E | L Arginine | 0,2 |
| E | Eau purifiée | 1,4 |
| F | Acide salicylique | 0,4 |
| F | Alcool agricole 96 % | 10,00 |
| G | Eau purifiée | 4,7 |
| G | Hydroxyde de sodium | 0,3 |
| H | PF pamplemousse E 9509056/07 | 0,3 |
| I | Eau purifiée | 8,58 |
| J | Caféine | 2,0 |
| K | Sol. Col. FDC bleu N1 0,1% sans conservateur | 0,640 |
| K | Sol. Col. FDC yellow 5 à 0,1 % dans MP DIOL | 0,440 |
| L | Bodyfit | 2,5 |
| L | Extrait de caroube selon l'exemple 1 | 2,00 |

### 2 Crème amincissante 2

| ***Phase*** | ***Composant*** | **% *massique*** |
|---|---|---|
| A | Eau purifiée | 49,615 |
| A | Hydroxyde de sodium | 0,110 |
| A | Methylpropane diol | 4,00 |
| B | Acide salicylique | 0,4 |
| C | Caféine | 1,5 |
| D | NA 4 EDTA | 0,1 |
| E | Synthalen K | 1,0 |
| F | Alcool cétylique 20 OE | 1,0 |
| F | Coco-caprylate caprate | 9,00 |
| F | Alcool cétylique | 2,00 |
| F | Beurre de karité 100 % veg. | 2,00 |
| F | Cire d'abeille 100 % naturelle | 2,00 |
| F | Acetate de tocophérol | 0,5 |
| F | Alcool stéarylique OV | 1,00 |
| F | Diméthicone 20CST | 2,00 |
| G | Hydroxyde de sodium | 0,350 |
| G | Eau purifiée | 2,00 |
| H | Sulfate de dextran | 0,5 |
| H | Extrait de caroube selon l'exemple 1 | 2,00 |
| H | Eau purifiée | 3,00 |
| I | Rutine | 0,05 |
| I | Propylène glycol | 4,00 |
| J | Alcool agricole 96 % | 11,00 |
| J | Sol. Col. FDC bleu N1 0,1% sans conservateur | 0,275 |
| J | PF revitalisant TER G111 25975 | 0,3 |

## Revendications

1. Utilisation cosmétique de l'association d'un extrait peptidique de germe de caroube (*Ceratonia siliqua L.*) et de caféine, ou un de ses dérivés, en tant qu'agent actif amincissant.

2. Utilisation cosmétique selon la revendication 1, dans laquelle l'extrait de germe de caroube contient entre 1,5 et 3,5 g/l de composés de nature peptidique.

3. Utilisation cosmétique selon l'une des revendications 1 ou 2, dans laquelle l'extrait de germe de caroube est extrait peptidique dans lequel les composés de nature peptidique ont un poids moléculaire inférieur à 5 kDa.

4. Utilisation cosmétique selon l'une des revendications précédentes, dans laquelle l'association d'un extrait peptidique de germe de caroube (*Ceratonia siliqua L.*) et de caféine, ou un de ses dérivés, provoque une augmentation de l'expression des aquaglycéroporines et plus particulièrement de l'aquaglycéroporine 7.

5. Utilisation cosmétique selon l'une des revendications précédentes, dans laquelle l'association d'un extrait peptidique de germe de caroube (*Ceratonia siliqua L.*) et de caféine, ou un de ses dérivés, augmente la lipolyse et favorise le déstockage des lipides et la sortie du glycérol des adipocytes.

6. Méthode de soin cosmétique comprenant l'application topique sur au moins une partie de la peau du corps ou du visage, d'un extrait peptidique de germe de caroube (*Ceratonia siliqua L.*) et de caféine, ou un de ses dérivés, dans une composition comprenant un milieu physiologiquement acceptable, pour obtenir un effet amincissant, et plus particulièrement pour diminuer les surcharges adipeuses localisées.

7. Méthode de soin cosmétique comprenant l'application topique sur au moins une partie de la peau du corps ou du visage, de l'association d'un extrait peptidique de germe de caroube (*Ceratonia siliqua L.*) et de caféine, ou un de ses dérivés, dans une composition comprenant un milieu physiologiquement acceptable, pour atténuer l'aspect en peau d'orange de la peau.

8. Méthode de soin cosmétique selon l'une des revendications 6 ou 7 dans laquelle l'extrait peptidique de germe de caroube, tel que décrit dans l'une des revendications 1 à 4, est présent à une concentration comprise entre 0,0001 % et 20 % du poids total de la composition, et préférentiellement à une concentration comprise entre 0,05 % et 5 % du poids total de la composition.

9. Méthode de soin cosmétique selon l'une des revendications 6 à 8 dans laquelle la caféine, ou un de ses dérivés, est présente à une concentration comprise entre 0,0001 % et 20 % du poids total de la composition, et préférentiellement à une concentration comprise entre 0,05 % et 5 % du poids total de la composition.

10. Méthode de soin cosmétique selon l'une des revendications 6 à 9 dans laquelle la composition comprend, en outre, au moins un agent actif additionnel choisi parmi la vitamine A, l'acide rétinoïque, le rétinol, la vitamine B3, la vitamine B5, la vitamine B6, la vitamine B12, la vitamine C, la vitamine E, la vitamine F, la vitamine H, la vitamine K, la vitamine PP, le coenzyme Q10, les inhibiteurs de métalloproteinase, les acides aminés, la carnitine, la carnosine, la taurine, les peptides naturels ou de synthèse, les extraits peptidiques végétaux, les extraits de levures, les extraits d'Artemia Salina, les phystostérols d'origine synthétique ou naturelle, l'acide salicylique, les oligosaccharides, les polysaccharides, les sucres aminés, les polyphénols, les flavonoïdes, les lipides, les phospholipides, l'AMP cyclique et ses dérivés, les agents activateurs de l'enzyme adénylate cyclase, les agents inhibiteurs de l'enzyme phosphodiestérase, la théine, la théophylline, la théobromine, la forskoline, l'esculine, les inhibiteurs d'ACE, l'extrait de dioscorea, l'extrait de guarana, l'extrait de lierre, l'extrait de fucus, les extraits d'algues tels que Palmata Palmaria, l'extrait hydrolysé de Prunella vulgaris ou l'extrait de sureau.

## Patentansprüche

1. Kosmetische Verwendung der Kombination eines Johannisbrotkeim-Peptidextrakts (*Ceratonia siliqua L*.) und von Koffein oder einem seiner Derivate als Schlankheitswirkstoff.

2. Kosmetische Verwendung nach Anspruch 1, wobei der Johannisbrotkeimextrakt zwischen 1,5 und 3,5 g/l an Verbindungen von Peptidnatur enthält.

3. Kosmetische Verwendung nach einem der Ansprüche 1 oder 2, wobei der Johannisbrotkeimextrakt Peptidextrakt ist, bei dem die Verbindungen von Peptidnatur ein Molekulargewicht von unter 5 kDA besitzen.

4. Kosmetische Verwendung nach einem der vorhergehenden Ansprüche, wobei die Kombination eines Johannisbrotkeim-Peptidextrakts (*Ceratonia siliqua L*.) und von Koffein oder einem seiner Derivate eine Steigerung der Expression der Aquaglyceroporine und insbesondere des Aquaglyceroporins 7 bewirken.

5. Kosmetische Verwendung nach einem der vorhergehenden Ansprüche, wobei die Kombination eines Johannisbrotkeim-Peptidextrakts (*Ceratonia siliqua L*.) und von Koffein oder einem seiner Derivate die Lipolyse steigert und den Abbau der Lipide und den Austritt des Glycerins aus den Adipozyten begünstigt.

6. Kosmetisches Pflegeverfahren, umfassend die topische Anwendung auf wenigstens einem Teil der Haut des Körpers oder des Gesichts, eines Johannisbrotkeim-Peptidextrakts (*Ceratonia siliqua L*.) und von Koffein oder einem seiner Derivate in einer Zusammensetzung, die ein physiologisch verträgliches Medium umfasst, um einen verschlankenden Effekt zu erhalten, und insbesondere, um die lokalisierten adipösen Überlastungen zu verringern.

7. Kosmetisches Pflegeverfahren, umfassend die topische Anwendung auf wenigstens einem Teil der Haut des Körpers oder des Gesichts, der Kombination eines Johannisbrotkeim-Peptidextrakts (*Ceratonia siliqua L*.) und von Koffein oder einem seiner Derivate in einer Zusammensetzung, die ein physiologisch verträgliches Medium umfasst, um das Orangenhautbild der Haut zu mildern.

8. Kosmetisches Pflegeverfahren nach einem der Ansprüche 6 oder 7, wobei der Johannisbrotkeim-Peptidextrakt, wie in einem der Ansprüche 1 bis 4 beschrieben, in einer Konzentration im Bereich zwischen 0,0001 % und 20 % des Gesamtgewichts der Zusammensetzung, und vorzugsweise in einer Konzentration im Bereich zwischen 0,05 % und 5 % des Gesamtgewichts der Zusammensetzung vorliegt.

9. Kosmetisches Pflegeverfahren nach einem der Ansprüche 6 bis 8, wobei das Koffein oder eines seiner Derivate in einer Konzentration im Bereich zwischen 0,0001 % und 20 % des Gesamtgewichts der Zusammensetzung, und vorzugsweise in einer Konzentration im Bereich zwischen 0,05 % und 5 % des Gesamtgewichts der Zusammensetzung vorliegt.

10. Kosmetisches Pflegeverfahren nach einem der Ansprüche 6 bis 9, wobei die Zusammensetzung unter anderem wenigstens einen zusätzlichen Wirkstoff umfasst, ausgewählt aus Vitamin A, Retinsäure, Retinol, Vitamin B3, Vitamin B5, Vitamin B6, Vitamin B12, Vitamin C, Vitamin E, Vitamin F, Vitamin H, Vitamin K, Vitamin PP, Coenzym Q10, Metalloproteinase-Inhibitoren, Aminosäuren, Carnitin, Carnosin, Taurin, natürlichen oder synthetischen Peptiden, pflanzlichen Peptidextrakten, Hefeextrakten, Artemia Salina-Extrakten, Phytosterolen synthetischen oder natürlichen Ursprungs, Salicylsäure, Oligosacchariden, Polysacchariden, Aminozuckern, Polyphenolen, Flavonoiden, Lipiden, Phospholipiden, zyklischem AMP und seinen Derivaten, Aktivatoren des Enzyms Adenylatzyklase, Inhibitoren des Enzyms Phosphodiesterase, Tein, Theophyllin, Theobromin, Forskolin, Aesculin, ACE-Inhibitoren, Dioscorea-Extrakt, Guarana-Extrakt, Efeu-Extrakt, Blasentang-Extrakt, Extrakt von Algen wie etwa Palmata Palmaria, hydrolysiertem Prunella vulgaris-Extrakt oder Holunder-Extrakt.

## Claims

1. Cosmetic use of the association of a peptide extract of carob germ *(Ceratonia siliqua L)* and caffeine, or one of its derivatives, as an active slimming agent.

2. Cosmetic use according to claim 1, in which the extract of carob germ contains between 1.5 and 3.5 g/l of peptide type compounds.

3. Cosmetic use according to one of claims 1 and 2, in which the extract of carob germ is a peptide extract in which the molecular weight of peptide type compounds is less than 5 kDa.

4. Cosmetic use according to one of the previous claims, in which the association of a peptide extract of carob germ *(Ceratonia siliqua L.)* and caffeine, or one of its derivatives, provokes an increase in the expression of aquaglyceroporins and more particularly paquaglyceroporin 7.

5. Cosmetic use according to one of the previous claims, in which the association of a peptide extract of carob germ *(Ceratonia siliqua L.)* and caffeine, or one of its derivatives, increases lipolysis and facilitates destocking of lipids and the release of glycerol from adipocytes.

6. Cosmetic care method including the topical application on at least part of the skin of the body or the face, of a peptide extract of carob germ *(Ceratonia siliqua L.)* and caffeine, or one of its derivatives, in a composition including a physiologically acceptable medium, to obtain a slimming effect and more particularly to reduce local excess adipose tissue.

7. Cosmetic care method including the topical application on at least part of the skin of the body or the face, of the association of a peptide extract of carob germ *(Ceratonia siliqua L.)* and caffeine, or one of its derivatives, in a composition including a physiologically acceptable medium, to attenuate the orange skin aspect of the skin.

8. Cosmetic care method according to either claim 6 or 7, in which the peptide extract of carob germ as described in one of claims 1 to 4 has a concentration of between 0.0001% and 20% of the total weight of the composition, and preferentially has a concentration of between 0.05% and 5% of the total weight of the composition.

9. Cosmetic care method according to one of claims 6 to 8, in which the concentration of caffeine or one of its derivatives is between 0.0001% and 20% of the total weight of the composition, and preferentially has a concentration between 0.05% and 5% of the total weight of the composition.

10. Cosmetic care method according to one of claims 6 to 9, in which the composition also comprises at least one additional active agent chosen from among vitamin A, retinoic acid, retinol, vitamin B3, vitamin B5, vitamin B6, vitamin B12, vitamin C, vitamin E, vitamin F, vitamin H, vitamin K, vitamin PP, coenzyme Q10, metalloproteinase inhibitors, amino acids, carnitine, carnosine, taurine, natural or synthetic peptides, plant peptide extracts, yeast extracts, Artemia Salina extracts, phystosterols with synthetic or natural origin, salicylic acid, oligosaccharides, polysaccharides, amino sugars, polyphenols, flavonoids, lipids, phospholipids, cyclic AMP and its derivatives, activating agents of the adenylate cyclase enzyme, inhibiting agents of the phosphodiesterase enzyme, thein, theophylline, theobromine, forskolin, esculin, ACE inhibitors, extract of dioscorea, extract of guarana, extract of ivy, extract of fucus, seaweed extracts such as Palmata Palmaria, hydrolysed extract of Prunella vulgaris or extract of elderberry.
